# EUROPÄISCHE PATENTANMELDUNG

(11) **EP 3 932 434 A2**
(43) Veröffentlichungstag der Anmeldung: **05.01.2022**
(21) Anmeldenummer: 21160605.8
(22) Anmeldetag: 04.03.2021
(51) Int. Cl.: A61L 2/04, A61L 2/238

(54) **SELBST-DESINFIZIERENDER GEGENSTAND**

(30) Priorität: 14.05.2020 DE 102020206087
(71) Anmelder: FRAUNHOFER-GESELLSCHAFT zur Förderung der angewandten Forschung e.V., 80686 München (DE)
(72) Erfinder: STIER, Simon, 97082 Würzburg (DE)
(74) Vertreter: Pfenning, Meinig & Partner mbB

(57) **Zusammenfassung**

Die Erfindung betrifft einen selbst-desinfizierenden Gegenstand mit einer zur Berührung mit einem menschlichen Körperteil vorgesehenen Kontaktfläche eines Substrats, wobei die Kontaktfläche zumindest bereichsweise beheizbar ist.

## Beschreibung

Die Erfindung betrifft einen selbst-desinfizierenden Gegenstand mit einer zur Berührung mit einem menschlichen Körperteil vorgesehenen Kontaktfläche eines Substrats, wobei die Kontaktfläche zumindest bereichsweise beheizbar ist.

Ein wesentlicher Hauptübertragungsweg von Krankheitserregern ist die Tröpfcheninfektion über Aerosole, die etwa beim Husten entstehen. Viruspartikel bleiben jedoch auch auf Metallen, Kunststoffen oder Glas je nach Bedingungen bis zu mehreren Tagen infektiös, Bakterien, insbesondere in ihrer persistenten Form (Sporen), sogar noch wesentlich länger. Über eine Schmierinfektion kann es daher auch auf diesem Weg zu einer Übertragung kommen, selbst wenn die betroffenen Personen nie direkt Kontakt hatten. Neben eigenverantwortlichen Maßnahmen wie Händewaschen und Vermeiden von Kontakt mit den Schleimhäuten ist eine regelmäßige Desinfizierung oft berührter Objekte wie Türgriffe, Handläufen oder Bedienoberflächen (Schalter, Stechuhren, etc.) essentiell. Noch wichtiger sind derartige Maßnahmen in medizinischen Einrichtungen, in den neben Viruserkrankung auch multirestente Bakterien ein ernstes Problem darstellen. Das regelmäßige manuelle Desinfizieren der Oberflächen ist jedoch mit erheblichem personellen Aufwand verbunden, zudem besteht bei chemischen Wirkstoffe die Gefahr einer Resistenzbildung.

Aus dem Stand der Technik ist die Ausstattung der Oberflächen mit antibakteriell und/oder antiviral wirkenden Stoffen wie etwa Kupfer oder Silber bekannt. Das Wirkspektrum solcher Stoffe ist jedoch eingeschränkt und kann durch Verschleiß wie etwa Korrosion, Abnutzung und Wirkstoffverarmung auch zeitlich begrenzt sein.

Alternativ zu passiven Schutzschichten sind auch zahlreiche aktive Systeme entwickelt worden. Solche aktiven Systeme sind aus EP 3 118 395 A2, US 2014/0137369 A1, US 2010/0140499 A1 und DE 20 2014 006 908 U1. Hieraus gehen Anwendungen hervor, bei denen Desinfektionsmittel automatisch oder mechanisch freigesetzt werden oder Oberflächen von Gegenständen mit UV-Licht bestrahlt werden. Dabei wird jedoch nur ein Teil der Oberfläche der Gegenstände behandelt oder es ist ein aufwändiger und potentiell fehleranfälliger Mechanismus bzw. erhebliche bauliche Änderungen am Gegenstand erforderlich. Im Allgemeinen lassen sich diese Konzepte auch nicht auf andere Halte- und Bedienoberflächen übertragen.

Eine weitere aus dem Stand der Technik bekannte Lösung ist die Wärmebehandlung zur Desinfizierung. So beschreiben WO 1995/019216, EP 1 438 263 A1, WO 2005/079948 und DE 4 206 901 A1 Vorrichtungen zur thermischen Desinfizierung von Elementen von Flüssigkeitskreisläufen. Dabei werden jedoch separate Heizelemente verwendet bzw. innere Oberflächen beheizt.

Beheizbare Oberflächen sind bisher vor allem für Komfortfunktionen im Auto wie etwa Sitz- oder Lenkradheizungen etabliert. Diese basieren jedoch auf Heizdrähten, Spulen oder Lacken, die den gesamten Volumenkörper erhitzen. Daher ist der Aufheizvorgang sehr langsam, energieintensiv und in der Regel auf niedrige Temperaturen beschränkt. Zudem können derartige Komponenten nicht auf bestehenden Oberflächen nachgerüstet werden. Beispiele für beheizte Oberflächenelemente im Fahrzeugbereich sind aus US 7,291,815 B2 (Komposit-Vereisungsschutz für Flugzeuge) und DE 10 2012 207 708 A1 (Heizlack für Fahrzeuge) bekannt. Diese sind jedoch zum Enteisen oder Erwärmen des Innenraums gedacht.

Die DE 10 2009 016 370 A1 beschreibt ein Bedienelement mit beheizter Kontaktfläche um diese zu desinfizieren. Das Heizelement ist jedoch separat von der Kontaktfläche ausgeführt. Die US 2016/0317684 A1 beschreibt eine beheizbare, selbstdesinfizierende Oberfläche, wobei die Energiezufuhr über elektromagnetische Wellen realisiert ist.

Ausgehend hiervon war es Aufgabe der vorliegenden Erfindung, die Oberfläche eines Gegenstands mit einer selbst-desinfizierenden Beschichtung zu versehen, um ein einfach zu realisierende und automatisierte Desinfektion dieser Gegenstände ermöglicht.

Diese Aufgabe wird durch den selbst-desinfizierenden Gegenstand mit den Merkmalen des Anspruchs 1 gelöst. Die weiteren abhängigen Ansprüche zeigen vorteilhafte Weiterbildungen auf.

Erfindungsgemäß wird ein selbst-desinfizierender Gegenstand mit einer zur Berührung mit einem menschlichen Körperteil vorgesehenen Kontaktfläche des Gegenstands bereitgestellt, wobei die Kontaktfläche zumindest bereichsweise ist. Dies wird dadurch erreicht, dass zumindest in Bereichen der Kontaktfläche mindestens eine Desinfektionsbeschichtung angeordnet ist. Diese Desinfektionsbeschichtung enthält mindestens eine elektrisch leitfähige Schicht, die zur resistiven Beheizung der mindestens einen elektrisch leitfähigen Schicht über mindestens zwei elektrische Kontaktierungen mit mindestens einer Steuerelektronik verschaltet ist.

Unter Desinfizierung bzw. Desinfektion ist im Rahmen der vorliegenden Erfindung generell eine Reduzierung der Anzahl von Krankheitserregern zu verstehen. Im Speziellen umfasst dies die Desinfektion im eigentlichen Sinne, d.h. eine Reduktion der Krankheitserreger um mindestens den Faktor 10⁻⁵, sowie eine Sterilisation, d.h. eine Reduktion der Krankheitserreger um mindestens den Faktor 10⁻⁷.

Grundsätzlich kommen alle menschlichen Körperteile für den Kontakt mit dem Gegenstand in Betracht, wobei es sich vorzugsweise um Körperteile unterschiedlicher Menschen handelt, da für solche Gegenstände eine Desinfektion besonders wichtig ist, um Ansteckungen zwischen mehreren Menschen zu vermeiden.

Mit der vorliegenden Erfindung sind die folgenden Vorteile verbunden:
- die Desinfizierung ist wirksam gegen alle Krankheitserreger (Algen, Bakterien, Parasiten, Pilze, Prionen, Protisten, Viren und Viroide)
- eine Abnutzung oder ein Verschleiß der desinfizierenden Beschichtung kann vermieden werden
- die erfindungsgemäße Desinfizierung ist wartungsfrei
- die erfindungsgemäße Desinfizierung kann vollautomatisch ohne Personaleinsatz erfolgen
- der Gegenstand selbst muss (abgesehen von der verwendeten Desinfektionsbeschichtung) baulich nicht verändert werden

Vorzugsweise wird der Zeitpunkt, die Dauer und die Temperatur der Beheizung durch die Steuerelektronik so gewählt, dass die bestimmungsgemäße Benutzung des Gegenstands weiterhin gegeben ist und eine ausreichend hohe Anzahl von Krankheitserregern auf der Oberfläche unschädlich gemacht werden, um die Übertragung solcher Erreger über die Oberfläche gegenüber einer nicht beheizten Variante der Oberfläche zu verringern. In längeren Nutzungspausen (etwa nachts) kann zudem eine höhere (etwa > 120°C) und längere Erhitzung vorgenommen werden um z. B. auch hitzeresistente Sporen unschädlich zu machen.

Eine bevorzugte Ausführungsform sieht vor, dass die Steuerelektronik mindestens einen Temperatursensor und/oder mindestens eine Temperaturregelung aufweist, wobei die Temperaturregelung bevorzugt für eine Oberflächentemperatur des Gegenstands von mindestens 70°C, besonders bevorzugt mindestens 120°C ausgelegt ist und/oder Betriebszustände erreicht werden, bei denen die Oberflächentemperatur zumindest bereichsweise mindestens 70°C, besonders bevorzugt mindestens 120°C beträgt.

Für die Regelung der Heizleistung bzw. erzielten Oberflächentemperatur durch die Steuerelektronik können übliche spannungsbasierte (Regelung des Stroms über die Spannung) oder zeitbasierte Verfahren (Regelung der Leistung über An- und Abschaltdauer, vgl. Pulsweitenmodulation).

Es ist bevorzugt, dass die mindestens eine Desinfektionsbeschichtung zwischen der vom Gegenstand abgewandten Oberfläche und der davon ausgehend nächsten (obersten) elektrisch leitfähigen Schicht mindestens eine elektrisch isolierende Schicht und/oder zwischen der zum Gegenstand gewandten Oberfläche und der davon ausgehend nächsten (untersten) elektrisch leitfähigen Schicht mindestens eine thermisch isolierende Schicht aufweist

Die mindestens eine elektrisch leitfähige Schicht besteht vorzugsweise aus einem der folgenden Materialien oder Kombinationen hiervon oder enthält diese:
- Metalle, insbesondere ausgewählt aus der Gruppe bestehend aus Kupfer, Aluminium, Gold, Silber, Zinn, Nickel, Eisen, Zink und Legierungen hiervon,
- Kohlenstoffmodifikationen, insbesondere ausgewählt aus der Gruppe bestehend aus Graphit, Graphen, Kohlenstofffasern, Kohlenstoffnanoröhren, Ruß und Mischungen hiervon,
- elektrisch leitfähigen Keramikwerkstoffen, insbesondere ausgewählt aus der Gruppe bestehend aus SiC, ZrC, TiC, WC, TiN, ZrN, TiB₂, ZrB₂, TiO, TiSi₂ oder MoSi₂,
- elektrisch leitfähige Partikel aufweisende Polymermaterialen, wobei die elektrisch leitfähigen Partikel bevorzugt ausgewählt sind aus der Gruppe bestehend aus Pulvern, Nanodrähten, Flakes oder Dendriten der genannten Metalle, Pulver der zuvor genannten Keramikwerkstoffen und der zuvor genannten Kohlenstoffmodifikationen.

Die mindestens eine elektrisch isolierende Schicht besteht vorzugsweise aus einem der folgenden Materialien oder Kombinationen hiervon oder enthält diese:
- thermisch leitfähige Materialen, insbesondere ausgewählt aus der Gruppe bestehend aus Bornitrid, Aluminiumoxid und Aluminiumnitrid
- Polymermaterialien enthaltend Partikel aus den zuvor genannten thermisch leitfähigen Materialen
- Polymermaterialien enthaltend Partikel aus den zuvor genannten elektrisch leitfähigen Materialen, wobei deren Volumengehalt unterhalb der Perkolationsschwelle liegt

Die mindestens eine thermisch isolierende Schicht besteht vorzugsweise aus einem der folgenden Materialien oder Kombinationen hiervon oder enthält diese:
- thermisch isolierenden Materialen, insbesondere ausgewählt aus der Gruppe bestehend aus Silicatkeramiken, Silicon, Polymethylmethacrylat, Polystyrol, Polyvinylchlorid, Glas- oder Mineralwolle
- Polymermaterialien enthaltend Partikel aus den zuvor genannten thermisch isolierenden Materialen,
- Poren aufweisende Materialien, insbesondere aufgeschäumte Materialen
- Hohlkörper aufweisende Materialien insbesondere Polymermaterialien, wobei die Hohlkörper insbesondere Glashohlkugeln oder Mikrohohlkugeln mit Polymerhülle sind.

Dabei ist es bevorzugt, dass die genannten Polymermaterialien ausgewählt sind aus der Gruppe bestehend aus Silicon, anorganisch-organischen Hybridpolymere wie organisch hoch modifizierten Polysiloxanen, Fluorsilicon, Polyurethan (PUR), Ethylen-Propylen-Dien-Kautschuk, Poly-Chlorbutadien (CR), Acrylnitril-Butadien (NBR), Polyesterharz, Epoxidharz, Acrylatharz, Phenolharz, Aminoharz, Polyoxymethylene (POM), Polypropylen (PP), Acrylnitril-Butadien-Styrol-Copolymer (ABS), Polycarbonat (PC), Polymethylmethacrylat (PMMA).

Eine bevorzugte Ausführungsform sieht vor, dass die mindestens eine elektrisch leitfähige Schicht eine einheitliche oder lokal variierende Schichtdicke im Bereich von 0,1 bis 1.000 µm, bevorzugt von 1 bis 100 um aufweist.

Die mindestens eine elektrisch isolierende Schicht weist vorzugsweise eine einheitliche oder lokal variierende Schichtdicke im Bereich von 0,1 bis 500 µm, bevorzugt von 1 bis 10 um auf.

Es ist bevorzugt, dass die mindestens eine thermisch isolierende Schicht eine einheitliche oder lokal variierende Schichtdicke im Bereich von 0,05 bis 10 mm, bevorzugt von 0,1 bis 1 mm aufweist.

Dabei ist es bevorzugt, dass die Wärmekapazität der mindestens einen elekrisch leitfähigen Schicht sehr gering, z.B. durch geeignete Materialauswahl und geringe Schichtdicken, während die Isolation durch die mindestens eine thermisch isolierende Schicht zum Gegenstand sehr hoch gehalten wird, so dass wenig Energie zum Erreichen und Halten der Zieltemperatur notwendig ist und bei einem (versehentlichem) Kontakt mit einem menschlichen Körperteil keine Verbrennungen auftreten.

In einer vorteilhaften Ausführung ist weiterhin der Emissionsgrad der obersten, vom Gegenstand wegzeigenden Schicht klein, um geringe Abstrahlverluste zu erreichen, und die Wärmeleitfähigkeit einer mindestens einen elektrisch isolierenden Schichten groß, um eine gleichmäßige Erwärmung zu erreichen.

In einer vorteilhaften Ausführung ist weiterhin die Schichtdicke der mindestens einen elektrische leitfähigen Schicht so an die Geometrie des Gegenstands bzw. des Substrats angepasst, dass eine gleichmäßige Erwärmung auftritt, z. B. indem bei einer Verjüngung der mindestens einen elektrisch leitfähigen Schicht die Schichtdicke erhöht wird. Alternativ kann dies auch durch eine Strukturierung der mindestens einen elektrisch leitfähigen Schicht, z. B. in Form einer Zerlegung derselben in einzelne Segmente, erfolgen.

Eine weitere bevorzugte Ausführungsform sieht vor, dass die mindestens eine elektrisch leitfähige Schicht mindestens zwei elektrisch voneinander isolierte Segmente aufweist die über separate elektrische Kontakte mit einer Steuerelektronik verbunden sind.

Es ist bevorzugt, dass mindestens eine der elektrisch leitfähigen Schichten als resistives, kapazitives oder induktives Sensorelement zur Messung von Abstand, Temperatur, Druck, Dehnung, Kraft, Scherung, Biegung oder Benetzung der Kontaktfläche ausgebildet ist oder als Näherungssensor für Lebewesen dient. Dies kann dazu dienen um eine menschliche Präsenz zu erkennen und zu lokalisieren und entsprechend zu reagieren (Beheizung abschalten, Warnsignal geben). Zudem können durch eine Auswertung der Impedanz z. B. vorhandene Bio-Filme oder eine erfolgte manuelle Reinigung mit flüssigen Reinigungsmitteln festgestellt werden.

Die mindestens eine Desinfektionsbeschichtung weist vorzugsweise eine Transmission von mindestens 10 %, bevorzugt 20 % auf. Durch solch eine transparente Desinfektionsbeschichtung, insbesondere durch transparenten elektrisch leitfähige Schichten, können die Gegenstände damit visuell unverändert bleiben.

Eine weitere bevorzugte Ausführungsform sieht vor, dass die mindestens eine Desinfektionsbeschichtung mindestens ein Leuchtelement aufweist. So können z.B. Leuchtelemente, wie eine LED, für ein visuelles Feedback integriert werden.

Vorzugsweise ist die Desinfektionsbeschichtung elastisch und weist besonders bevorzugt eine Elastizität von mindestens 5 %, besonders bevorzugt mindestens 20 % auf.

Der erfindungsgemäße Gegenstand ist vorzugsweise verformbar, z. B. ein Schaumstoff, Polster oder Kissen. Durch eine elastische Desinfektionsbeschichtung, insbesondere einer elastischen elektrisch leitfähigen Schicht, können so auch verformbare Objekte wie etwa Polster ausgerüstet werden.

Es ist bevorzugt, dass der Gegenstand ausgewählt ist aus der Gruppe bestehend aus
- Manuell bedienbare Geräte bzw. mechanische Halte- und Bedienvorrichtungen, insbesondere Türklinken, Handläufe, Haltestangen
- Schalt- oder Bedienelemente, insbesondere Lichtschalter, Türöffner, Aufzugsbedienelemente,
- medizinischen Geräten, insbesondere manuell bedienbare medizinische Geräte und Werkzeuge, Patientenliegen, Operationstische,
- Sportgeräte, insbesondere Fitnessgeräte.

Ein Ausführungsbespiel ist eine erfindungsgemäß ausgestattete Türklinke. Diese wird in geeigneten Zeitspannen erhitzt um die Oberfläche zu desinfizieren. Die Desinfizierung erfolgt beispielsweise periodisch oder wenn die Steuerelektronik durch geeignete Sensorik eine Nutzungspause feststellt (etwa durch Bewegungsmelder oder Näherungssensorik).

Ein weiteres Ausführungsbeispiel ist ein erfindungsgemäß ausgestatteter Operationstisch. Dieser kann zwischen zwei Operationen manuell oder automatisch ausgelöst zur Desinfektion erhitzt werden. Weiterhin kann auch eine Temperierung der Oberfläche für den Patientenkomfort erzielt werden.

Anhand der nachfolgenden Figuren soll der erfindungsgemäße Gegenstand näher erläutert werden, ohne diesen auf die hier gezeigten spezifischen Ausführungsformen einschränken zu wollen.
- Fig. 1: zeigt eine erste erfindungsgemäße Ausführungsform
- Fig. 2: zeigt eine weitere erfindungsgemäße Ausführungsform für einen flachen Gegenstand
- Fig. 3: zeigt eine weitere erfindungsgemäße Ausführungsform für einen flachen Gegenstand mit segmentierter elektrisch leitfähiger Schicht
- Fig.4: zeigt eine weitere erfindungsgemäße Ausführungsform für einen zylindrischen Gegenstand
- Fig.5: zeigt eine weitere erfindungsgemäße Ausführungsform für einen zylindrischen Gegenstand
- Fig. 6: zeigt eine weitere erfindungsgemäße Ausführungsform für eine Türklinke

Für alle Figuren werden folgende Bezugszeichen verwendet:
- 1: beschichteter Gegenstand
- 2: thermisch isolierende Schicht
- 3: elektrische leitfähige Schicht
- 3a,3b: Segmente der elektrische leitfähigen Schicht
- 4: elektrisch isolierende Schicht
- 4a,4b: Segmente der elektrische isolierenden Schicht
- 5, 5a, 5b: elektrische Kontaktierung(en)
- 6: Steuerelektronik mit Stromquelle
- 7: Krankheitserreger auf der Kontaktoberfläche
- 8: Türbeschlag

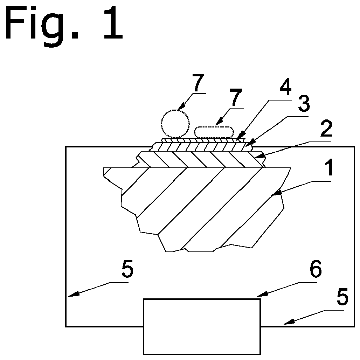

Wie in Fig. 1 dargestellt umfasst eine erfindungsgemäße Ausführungsform eine resistiv beheizbare Beschichtung für ein die Oberfläche eines exponierten Gegenstands 1. Diese besteht in einer vorteilhaften Ausführung aus einer thermisch und ggf. auch elektrisch isolierenden Schicht 2, einer elektrisch leitfähigen Schicht 3 und einer elektrisch isolierenden und ggf. thermisch leitfähigen Schicht 4. Eine Steuerelektronik 6 leitet bei Bedarf über mindestens zwei elektrische Kontakte 5 elektrischen Strom durch die mittlere Schicht 3 um diese resistiv zu heizen. Bei einer ausreichend hohen Oberflächentemperatur (in der Regel > 70°C) werden Viren, Bakterien, Bakteriensporen, Pilze, Pilzsporen und andere Erreger 7 unschädlich gemacht.

Für die Regelung der Heizleistung bzw. erzielten Oberflächentemperatur durch die Steuerelektronik können übliche spannungsbasierte (Regelung des Stroms über die Spannung) oder zeitbasierte Verfahren (Regelung der Leistung über An- und Abschaltdauer, vgl. Pulsweitenmodulation).

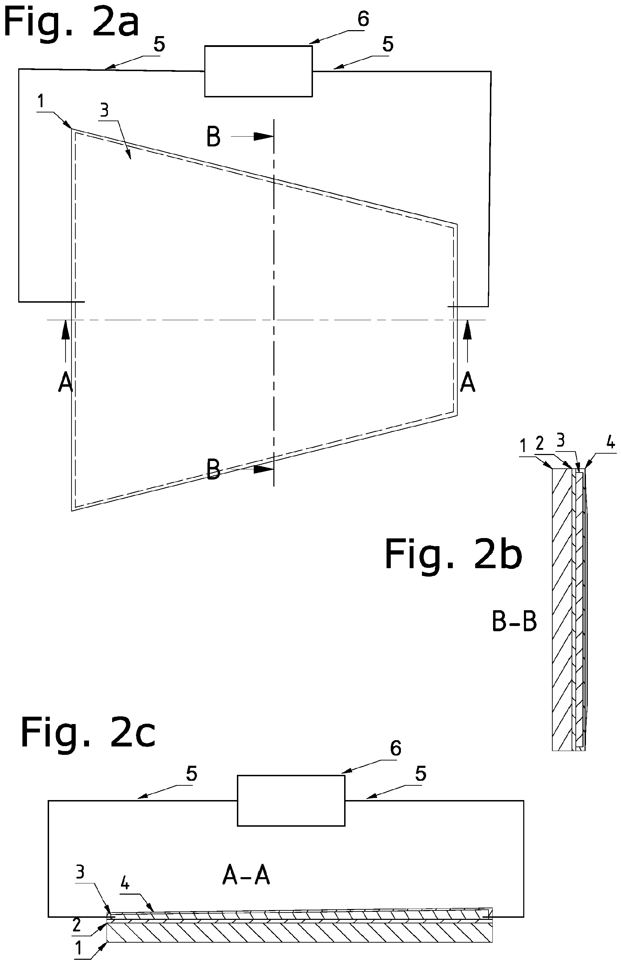

Eine weitere vorteilhafte Ausführung für einen flächigen Gegenstand ist in Fig. 2 dargestellt. Fig. 2a. zeigt eine Übersicht eines beispielhaften, unregelmäßig geformten Flächenstücks. Fig. 2b zeigt die in Fig. 2a definierte Schnittansicht (B-B). Fig. 2c zeigt die in Fig. 2a definierte Schnittansicht (A-A). Hier ist ersichtlich, das die Schichtdicke der leitfähigen Schicht 3 mit einem Schichtdickengradienten versehen wurde um eine gleichmäßige Erwärmung zu erzielen, d. h. in diesem Fall, dass bei Verjüngung der leitfähigen Schicht 3 deren Schichtdicke erhöht ist. Ohne diesen Schichtdickengradienten, d. h. bei einer homogenen Schichtdicke käme es bei konstanter spezifischen Leitfähigkeit des für die leitfähige Schicht 3 verwendeten Materials zu einer stärkeren Aufheizung am schmäleren Ende des beschichteten Gegenstandes 1, da hier der elektrische Spannungsabfall durch den größeren elektrischen Widerstand höher ist und damit auch die abgegebene Leistung.

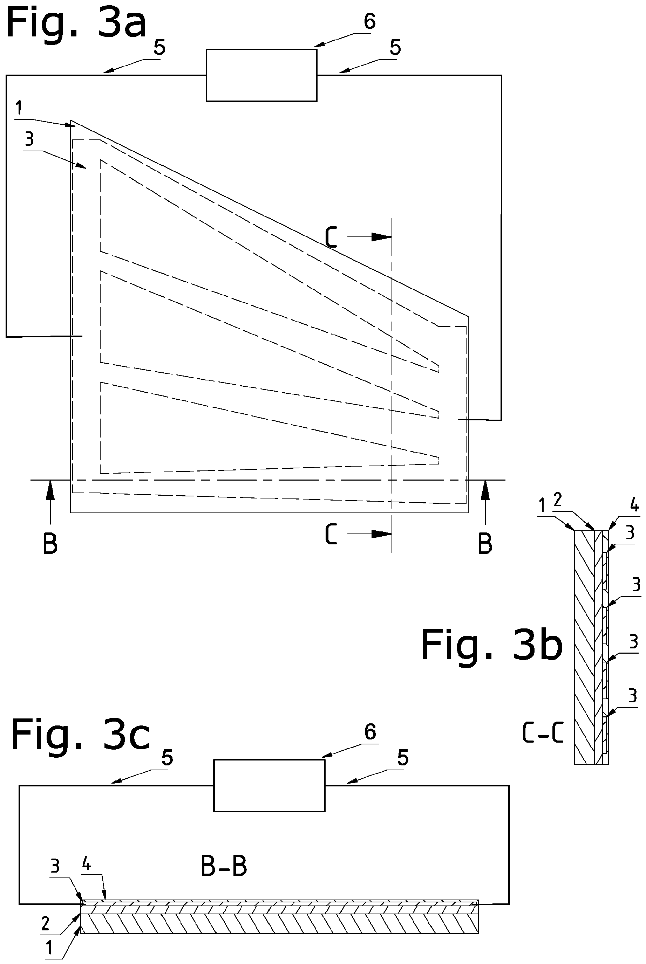

Fig. 3 zeigt eine alternative vorteilhafte Ausführung, bei der die gleichmäßige Erwärmung durch eine Strukturierung der leitfähigen Schicht erzielt wird. Fig. 3a. zeigt eine Übersicht eines bespielhaften, unregelmäßig geformten Flächenstücks. Hier ist ersichtlich, dass die elektrisch leitfähige Schicht 3 in einzelne Segmente unterteilt ist, deren Breite sich mit der Vergrößerung der Breite des beschichteten Gegenstandes 1 verringert. Ohne diese Strukturierung, d. h. bei einer vollflächigen Beschichtung mit homogener Schichtdicke käme es bei konstanter spezifischen Leitfähigkeit des für die leitfähige Schicht 3 verwendeten Materials zu einer stärkeren Aufheizung am schmäleren Ende des beschichteten Gegenstandes 1, da hier der elektrische Spannungsabfall durch den größeren elektrischen Widerstand höher ist und damit auch die abgegebene Leistung. Die Kontaktierung der Segmente mit der Steuerelektronik 6 könnte auch über separate elektrische Kontakte erfolgen. Dies hätte den Vorteil, dass die Regelung der Heizleistung pro Segment ebenfalls separat erfolgen kann. Fig. 3b zeigt die in Fig. 3a definierte Schnittansicht (C-C). Fig. 3c zeigt die in Fig. 3a definierte Schnittansicht (B-B).

Mit den in Fig. 2 und 3 gezeigten vorteilhaften Ausführungen kann z. B. ein Operationstisch ausgestattet werden. Dieser kann zwischen zwei Operationen manuell oder automatisch ausgelöst zur Desinfektion erhitzt werden. Zusätzlich kann auch eine Temperierung der Oberfläche für den Patientenkomfort erzielt werden. Derart können weiterhin auch andere medizinische Instrumente sowie flächige Bedienelemente in Gebäuden wie etwa Lichtschalter ausgestattet werden.

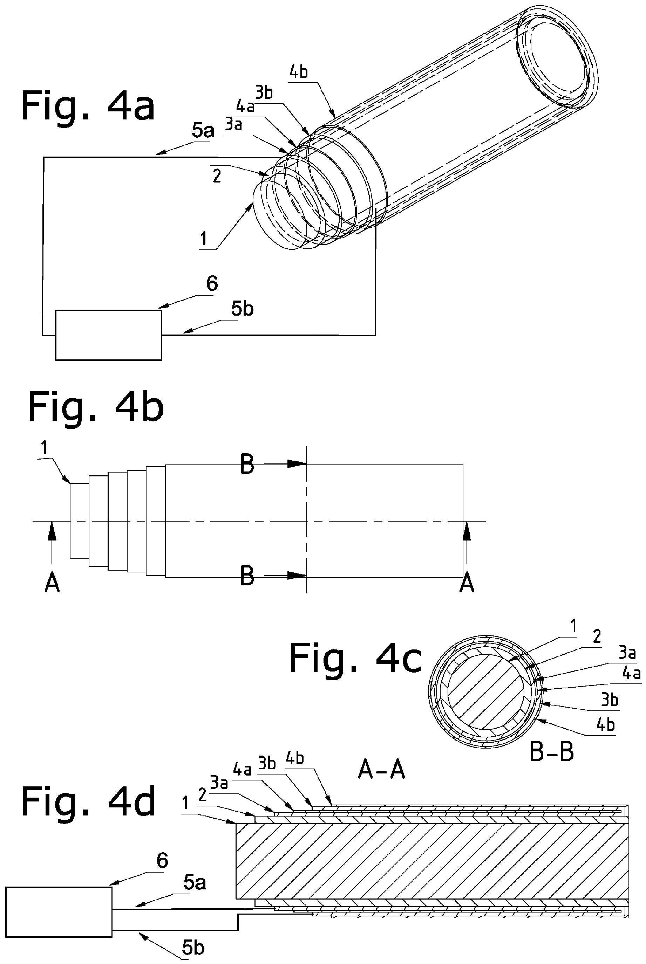

Fig. 4 zeigt eine weitere vorteilhafte Ausführung für einen zylindrischen Gegenstand. Fig. 4a zeigt eine Übersicht eines bespielhaften, hier der Übersicht wegen verkürzt dargestellten zylindrisches Gegenstandes. Die Länge ist grundsätzlich jedoch frei wählbar. Weiterhin dient auch stufenförmig entfernten Schichten am Ende lediglich dem besseren Verständnis. Die elektrisch leitfähige Schicht ist hierbei in zwei Schichten 3a und 3b aufgeteilt. Diese sind von einer weiteren elektrisch isolierenden Schicht 4a von einander elektrisch isoliert. Die Verbindung mit der Steuerelektronik erfolgt über die elektrischen Kontakte 5a bzw. 5b. Wie zuvor befindet sich auch eine elektrisch isolierende Schicht an der Oberfläche 4b. In Fig. 4b ist die Seitenansicht mit der Definition der Schnitte (A-A) und (B-B) dargestellt. Fig. 4c zeigt die in Fig. 4b definierte Schnittansicht (B-B). Fig. 4d zeigt die in Fig. 4b definierte Schnittansicht (B-B). Hier ist ersichtlich, dass die separierten elektrisch leitfähigen Schichten 3a und 3b am Ende des beschichteten Gegenstands 1 mit einander verbunden sind und der Heizstromkreis damit geschlossen wird. Im Vergleich zu einer Ausführung mit nur einer elektrisch leitfähigen Schicht kann daher in vorteilhafter Weise der elektrische Anschluss 5 der Steuerelektronik 6 lediglich an einem Ende des Gegenstandes 1 erfolgen.

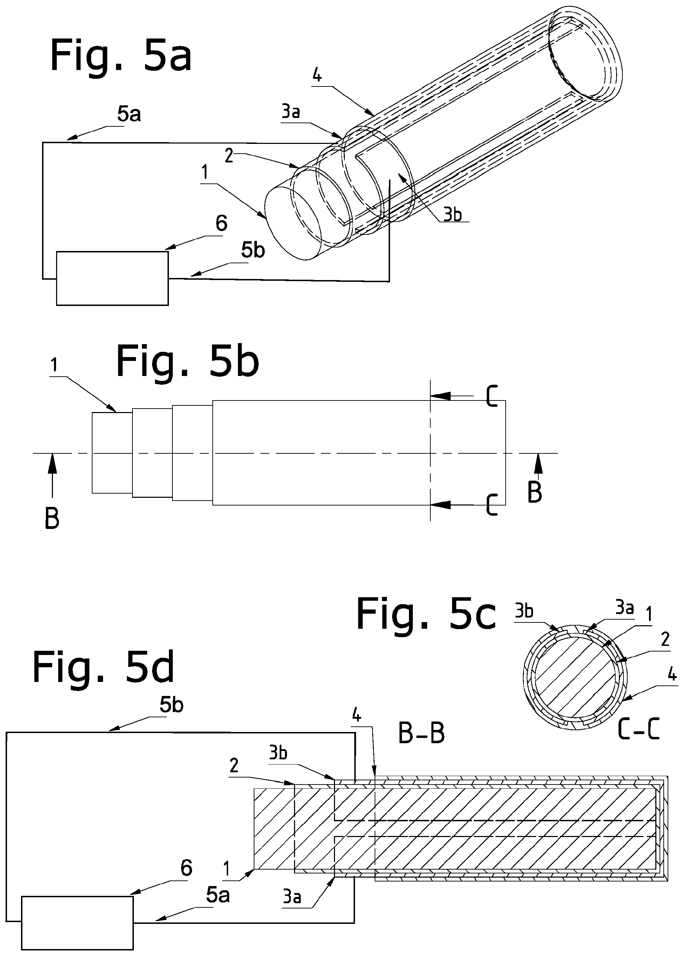

Fig. 5 zeigt eine alternative vorteilhafte Ausführung für einen zylindrischen Gegenstand. Fig. 5a zeigt eine Übersicht eines bespielhaften, hier der Übersicht wegen verkürzt dargestellten zylindrisches Gegenstandes. Die Länge ist grundsätzlich jedoch frei wählbar. Weiterhin dient auch stufenförmig entfernten Schichten am Ende lediglich dem besseren Verständnis. Die elektrisch leitfähige Schicht ist hierbei in zwei Teilschichten 3a und 3b aufgeteilt. Diese sind von der isolierenden Schicht 4 von einander elektrisch isoliert. Die Verbindung mit der Steuerelektronik erfolgt über die elektrischen Kontakte 5a bzw. 5b. In Fig. 5b ist die Seitenansicht mit der Definition der Schnitte (C-C) und (B-B) dargestellt. Fig. 5c zeigt die in Fig. 5b definierte Schnittansicht (C-C). Hier ist ersichtlich, dass die elektrisch leitfähigen Teilschichten 3a und 3b durch einen Spalt voneinander isoliert sind. Fig. 5d zeigt die in Fig. 5b definierte Schnittansicht (B-B). Hier ist ersichtlich, dass die elektrisch leitfähigen Teilschichten 3a und 3b am Ende des beschichteten Gegenstands 1 mit einander verbunden sind und der Heizstromkreis damit geschlossen wird. Im Vergleich zu einer Ausführung mit nur einer elektrisch leitfähigen Schicht kann daher in Vorteilhafter Weise der elektrische Anschluss 5 der Steuerelektronik 6 lediglich an einem Ende des Gegenstandes 1 erfolgen. Im Vergleich zur in Fig. 4 gezeigten Ausführungen werden dafür jedoch keine zusätzlichen Schichten benötigt.

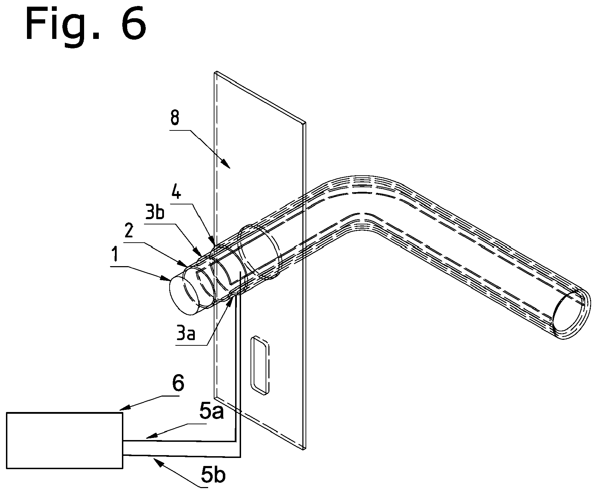

In Fig. 6 ist die in Fig. 5 gezeigte vorteilhafte Ausführung in Form einer Türklinke dargestellt. Diese kann durch die erfindungsgemäße Ausstattung in geeigneten Zeitspannen erhitzt um die Oberfläche zu desinfizieren. Die Desinfizierung erfolgt beispielsweise periodisch oder wenn die Steuerelektronik durch geeignete Sensorik eine Nutzungspause feststellt, etwa durch Bewegungsmelder oder Näherungssensorik. Allgemein kann eine zusätzliche Sensorik auch in die Beschichtung selbst, z. B. in Form einer zusätzlichen leitfähigen Schicht integriert werden, deren Kapazität, Induktivität und Widerstand erfasst wird. Zur Veranschaulichung ist in Fig. 6 auch der Türbeschlag 8 dargestellt.

Die in Fig. 4 und Fig. 5 gezeigten vorteilhaften Ausführungen können weiterhin für andere zylindrische Gegenstände wie etwa Handläufe, Haltegriffe und Haltestangen eingesetzt werden.

## Patentansprüche

1. Selbst-desinfizierender Gegenstand mit einer zur Berührung mit einem menschlichen Körperteil vorgesehenen Kontaktfläche des Gegenstands, wobei die Kontaktfläche zumindest bereichsweise selbstdesinfizierend ist, indem zumindest in Bereichen der Kontaktfläche mindestens eine Desinfektionsbeschichtung enthaltend mindestens eine elektrisch leitfähige Schicht, die zur resistiven Beheizung der mindestens einen elektrisch leitfähigen Schicht über mindestens zwei elektrische Kontaktierungen mit mindestens einer Steuerelektronik verschaltet ist, angeordnet ist.

2. Gegenstand nach Anspruch 1,
**dadurch gekennzeichnet, dass** die Steuerelektronik mindestens einen Temperatursensor und/oder mindestens eine Temperaturregelung aufweist, wobei die Temperaturregelung bevorzugt für eine Oberflächentemperatur des Gegenstands von mindestens 70°C, besonders bevorzugt mindestens 120°C ausgelegt ist.

3. Gegenstand nach einem der vorhergehenden Ansprüche,
**dadurch gekennzeichnet, dass** die mindestens eine Desinfektionsbeschichtung zwischen der vom Gegenstand abgewandten Oberfläche und der davon ausgehend nächsten elektrisch leitfähigen Schicht mindestens eine elektrisch isolierende Schicht und/oder zwischen der zum Gegenstand gewandten Oberfläche und der davon ausgehend nächsten elektrisch leitfähigen Schicht mindestens eine thermisch isolierende Schicht aufweist.

4. Gegenstand nach einem der vorhergehenden Ansprüche,
**dadurch gekennzeichnet, dass** die mindestens eine elektrisch leitfähige Schicht aus einem der folgenden Materialien oder Kombinationen hiervon besteht oder diese enthält:
• Metalle, insbesondere ausgewählt aus der Gruppe bestehend aus Kupfer, Aluminium, Gold, Silber, Zinn, Nickel, Eisen, Zink und Legierungen hiervon,
• Kohlenstoffmodifikationen, insbesondere ausgewählt aus der Gruppe bestehend aus Graphit, Graphen, Kohlenstofffasern, Kohlenstoffnanoröhren, Ruß und Mischungen hiervon,
• elektrisch leitfähigen Keramikwerkstoffen, insbesondere ausgewählt aus der Gruppe bestehend aus SiC, ZrC, TiC, WC, TiN, ZrN, TiB₂, ZrB₂, TiO, TiSi₂ oder MoSi₂,
• elektrisch leitfähige Partikel aufweisende Polymermaterialen, wobei die elektrisch leitfähigen Partikel bevorzugt ausgewählt sind aus der Gruppe bestehend aus Pulvern, Nanodrähten, Flakes oder Dendriten der genannten Metalle, Pulver der zuvor genannten Keramikwerkstoffen und der zuvor genannten Kohlenstoffmodifikationen .

5. Gegenstand nach einem der vorhergehenden Ansprüche,
**dadurch gekennzeichnet, dass** die mindestens eine elektrisch isolierende Schicht aus einem der folgenden Materialien oder Kombinationen hiervon besteht oder diese enthält:
• thermisch leitfähige Materialen, insbesondere ausgewählt aus der Gruppe bestehend aus Bornitrid, Aluminiumoxid und Aluminiumnitrid
• Polymermaterialien, bevorzugt enthaltend Partikel aus den zuvor genannten thermisch leitfähigen Materialen.
• Polymermaterialien enthaltend Partikel, bevorzugt sphärische Partikel, aus den zuvor genannten elektrisch leitfähigen Materialen, wobei deren Volumengehalt unterhalb der Perkolationsschwelle liegt

6. Gegenstand nach einem der vorhergehenden Ansprüche,
**dadurch gekennzeichnet, dass** die mindestens eine thermisch isolierende Schicht aus einem der folgenden Materialien oder Kombinationen hiervon besteht oder diese enthält:
• thermisch isolierenden Materialen, insbesondere ausgewählt aus der Gruppe bestehend aus Silicatkeramiken, Silicon, Polymethylmethacrylat, Polystyrol, Polyvinylchlorid, Glas- oder Mineralwolle
• Polymermaterialien enthaltend Partikel aus den zuvor genannten thermisch isolierenden Materialen,
• Poren aufweisende Materialien, insbesondere aufgeschäumte Materialen,
• Hohlkörper aufweisende Materialien, insbesondere Polymermaterialien, wobei die Hohlkörper insbesondere Glashohlkugeln oder Mikrohohlkugeln mit Polymerhülle sind.

7. Gegenstand nach einem der Ansprüche 4 bis 6,
**dadurch gekennzeichnet, dass** die Polymermaterialien ausgewählt sind aus der Gruppe bestehend aus Silicon, anorganisch-organischen Hybridpolymere wie organisch hoch modifizierten Polysiloxanen, Fluorsilicon, Polyurethan (PUR), Ethylen-Propylen-Dien-Kautschuk, Poly-Chlorbutadien (CR), Acrylnitril-Butadien (NBR), Polyesterharz, Epoxidharz, Acrylatharz, Phenolharz, Aminoharz, Polyoxymethylene (POM), Polypropylen (PP), Acrylnitril-Butadien-Styrol-Copolymer (ABS), Polycarbonat (PC), Polymethylmethacrylat (PMMA).

8. Gegenstand nach einem der vorhergehenden Ansprüche,
**dadurch gekennzeichnet, dass** die mindestens eine elektrisch leitfähige Schicht eine einheitliche oder lokal variierende Schichtdicke im Bereich von 0,1 bis 1000 µm, bevorzugt von 1 bis 100 µm und/oder die mindestens eine elektrisch isolierende Schicht eine einheitliche oder lokal variierende Schichtdicke im Bereich von 0,1 bis 500 µm, bevorzugt von 1 bis 10 µm und/oder die mindestens eine thermisch isolierenden Schicht eine einheitliche oder lokal variierende Schichtdicke im Bereich von 0,05 bis 10 mm, bevorzugt von 0,1 bis 1 mm aufweist.

9. Gegenstand nach einem der vorhergehenden Ansprüche,
**dadurch gekennzeichnet, dass** die beschichtete Kontaktfläche eine unregelmäßige Form, d. h. eine schmälere und eine breitere Seite aufweist, wobei mindestens eine der elektrisch leitfähigen Schichten im Mittel in Richtung der schmäleren Seite dicker und in Richtung der breiteren Seite dünner wird und/oder mindestens eine der elektrisch leitfähigen Schichten in mindestens einen Streifen aufgeteilt ist, der in Richtung der schmäleren Seite breiter und Richtung der breiteren Seite schmäler wird.

10. Gegenstand nach einem der vorhergehenden Ansprüche,
**dadurch gekennzeichnet, dass** die mindestens eine elektrisch leitfähige Schicht mindestens zwei elektrisch voneinander isolierte Segmente aufweist.

11. Gegenstand nach einem der vorhergehenden Ansprüche,
**dadurch gekennzeichnet, dass** mindestens eine der elektrisch leitfähige Schichten als resistives, kapazitives oder induktives Sensorelement zur Messung von Abstand, Temperatur, Druck, Dehnung, Kraft, Scherung, Biegung oder Benetzung der Kontaktfläche ausgebildet ist oder als Näherungssensor für Lebewesen dient.

12. Gegenstand nach einem der vorhergehenden Ansprüche,
**dadurch gekennzeichnet, dass** die mindestens eine Desinfektionsbeschichtung eine Transmission von mindestens 10 % und/oder mindestens ein Leuchtelement aufweist.

13. Gegenstand nach einem der vorhergehenden Ansprüche,
**dadurch gekennzeichnet, dass** die Desinfektionsbeschichtung elastisch ist und bevorzugt eine Elastizität von mindestens 5 %, besonders bevorzugt mindestens 20 % aufweist.

14. Gegenstand nach einem der vorhergehenden Ansprüche,
**dadurch gekennzeichnet, dass** der Gegenstand verformbar ist, insbesondere ein Schaumstoff, Polster oder Kissen.

15. Gegenstand nach einem der vorhergehenden Ansprüche,
**dadurch gekennzeichnet, dass** der Gegenstand ausgewählt ist aus der Gruppe bestehend aus
• Manuell bedienbare Geräte, insbesondere Türklinken,
• mechanische Halte- und Bedienvorrichtungen, insbesondere Handläufe, Haltestangen,
• Schalt- oder Bedienelemente, insbesondere Lichtschalter, Türöffner, Aufzugsbedienelemente,
• medizinischen Geräten, insbesondere manuell bedienbare medizinische Geräte und Werkzeuge, Patientenliegen, Operationstische,
• Sportgeräte, insbesondere Fitnessgeräte.
